# FASCICULE DE BREVET EUROPEEN

(11) **EP 1 560 534 B1**
(45) Date de publication et mention de la délivrance du brevet: **11.07.2007**
(21) Numéro de dépôt: 03761666.1
(22) Date de dépôt: 30.06.2003
(51) Int. Cl.: A61B 17/74

(54) **DISPOSITIF D OSTEOSYNTHESE EPIPHYSAIRE EN DEUX PARTIES, NOTAMMENT POUR CLOU OU PLAQUE**
ZWEITEILIGE VORRICHTUNG FÜR EPIPHYSÜRE OSTEOSYNTHESE, IM BESONDEREN FÜR EINEN NAGEL ODER EINE PLATTE
DEVICE FOR TWO-PART EPHYSEAL OSTEOSYNTHESIS, IN PARTICULAR FOR A NAIL OR A PLATE

(30) Priorité: 28.06.2002 FR 0208122
(43) Date de publication de la demande: 10.08.2005
(73) Titulaire: SARL GROUPE LEPINE, 69394 Lyon cedex 03 (FR); Langlais, Frantz, F-35200 Rennes (FR); Burdin, Philippe, 37320 Saint-Branchs (FR)
(72) Inventeur: LANGLAIS, Frantz, F-35200 Rennes (FR); GENTIL, Pascal, F-64300 Orthez (FR); BURDIN, Philippe, F-37320 Saint-Branchs (FR)
(74) Mandataire: Le Forestier, Eric
(86) Numéro de dépôt international: PCT/FR2003/002018
(87) Numéro de publication internationale: WO 2004/002342

(56) Documents cités:
- EP-A- 0 411 273
- FR-A- 2 668 360
- US-A- 2 121 193
- US-A- 4 776 330
- PATENT ABSTRACTS OF JAPAN vol. 2000, no. 05, 14 septembre 2000 (2000-09-14) & JP 2000 051224 A (MURASHIMA YOJI), 22 février 2000 (2000-02-22)

## Description

L'invention concerne les dispositifs d'ostéosynthèse notamment métaphysaire, par exemple pour le fémur distal, le tibia proximal ou encore l'humérus distal ou proximal, et plus particulièrement des vis céphaliques pour de tels sites d'ostéosynthèse, ou encore d'ostéosynthèse diaphysaire de type vis-plaque.

Dans le domaine des clous centro-médullaires permettant de traiter une grande variété de fractures notamment du fémur, on connaît un dispositif comportant une ou deux vis céphaliques passant au travers du clou dans sa partie proximale. De manière générale, le clou est essentiellement un tube cylindrique de révolution et la vis céphalique est insérée dans ledit clou dans sa partie supérieure ou partie proximale. Ceci impose que le diamètre maximal de la vis céphalique soit inférieur au diamètre du clou au niveau où est introduite ladite vis céphalique.

De ce fait, le dispositif présente :
- soit un diamètre de vis céphalique petit, diminuant par conséquence la stabilité et la tenue de celle-ci dans la tête fémorale ainsi que la rigidité de ladite vis ;
- soit un diamètre de clou important dans sa partie supérieure au moins, augmentant de fait l'alésage à réaliser dans l'os de manière à élargir le canal médullaire recevant le clou, ce qui a pour conséquence de fragiliser le fémur à cet endroit lors de la pose dudit clou.

Le document FR-2 668 360 décrit par ailleurs une vis céphalique en deux parties : elle comporte une partie formant tige et une partie formant tête adaptable sur une extrémité de la partie formant tige et présentant un filetage osseux sur son pourtour externe. La tête est vissée sur la tige. Ce dispositif, bien que résolvant en partie les problèmes précités, oblige à ce que la tige soit impérativement un cylindre de révolution afin de permettre l'assemblage de la tige avec la tête à travers le clou lors de la pose.

Comme cela est expliqué dans le FR-A 2 668 360 (page 3, dernier alinéa), il est ainsi possible d'utiliser un clou de diamètre réduit, non traumatisant pour l'os dans lequel il est implanté, tout en disposant d'un noyau fileté de grand diamètre apte à assurer un bon ancrage dans le tissu osseux.

Ce dispositif connu présente cependant certains inconvénients.

En premier lieu, l'assemblage de la tige avec le noyau se fait par vissage, ce qui est une opération relativement malaisée et parfois délicate pour le chirurgien.

En second lieu, du fait que la tige et l'orifice du clou sont cylindriques, ce qui est nécessaire pour permettre le vissage de la tige dans le noyau, la résistance de la tige à la flexion n'est pas optimale, et la résistance mécanique du clou est également affaiblie dans la zone traversée par ledit orifice.

Sont par ailleurs compris dans l'état de la technique les documents US-A-4 776 330 et EP-A-0 411 273.

Le document US-A-4 776 330 concerne un dispositif d'ostéosynthèse notamment métaphysaire, possédant un organe d'ancrage non fileté, mais expansible, apte à se dilater radialement à la manière d'une cheville de fixation murale. Cet organe d'ancrage est porté par une tige cylindrique, qui peut tourner librement soit dans l'alésage formé dans le canon d'une plaque diaphysaire, soit dans une pièce de montage destinée à être emmanchée sur un clou centromédullaire.

Le document EP-A-0 411 273 décrit un clou centromédullaire dont la partie proximale est percée d'une lumière allongée recevant une lame vrillée ; celle-ci possède une portion d'extrémité proximale à bord coupant, permettant sa pénétration dans l'os.

Aucun de ces documents ne se rapporte donc à un dispositif d'ostéosynthèse dont l'organe d'ancrage est un noyau fileté, apte à être mise en place par vissage dans l'os.

Le dispositif qui fait l'objet de l'invention est un dispositif d'ostéosynthèse, notamment métaphysaire, de même type général que celui de FR-A-2 668 360. Il comprend un clou centromédullaire solidaire d'une vis qui est composée de deux éléments distincts, à savoir une tige et un organe d'ancrage, ce dernier consistant en un noyau essentiellement cylindrique comportant un filetage externe apte à être mis en place par vissage dans l'os, tandis que la tige est adaptée pour être insérée dans un orifice traversant ménagé dans la partie proximale du clou.

L'invention se propose de résoudre les problèmes mentionnés plus haut en permettant une pose du dispositif par un geste opératoire plus simple, ainsi qu'une optimisation de la section de la tige, afin que celle-ci soit stable et résistante.

Ces objectifs sont atteints, conformément à l'invention, grâce au fait que :
a) ladite tige possède une partie proximale dont la portion d'extrémité a une forme tronconique, se rétrécissant vers son extrémité libre, ainsi qu'une partie distale de forme aplatie, à section allongée ;
b) ledit organe d'ancrage possède un orifice axial qui présente une section de forme tronconique, complémentaire de ladite portion d'extrémité de la partie proximale ;
c) ledit orifice traversant a la forme d'une lumière allongée, dont la grande direction est sensiblement parallèle à l'axe de la partie proximale du clou, et dont la section est sensiblement identique à celle de la partie distale de la tige, de sorte que cette dernière peut être reçue à coulissement dans ledit orifice, sans possibilité de rotation par rapport au clou centromédullaire ;
d) l'assemblage de la tige avec organe d'ancrage est réalisé par simple translation de la tige selon son axe longitudinal et engagement de sa portion d'extrémité dans l'orifice axial de l'organe d'ancrage, leur liaison mutuelle étant assurée par friction, par cônes Morse complémentaires.

Ainsi, lors de la pose du dispositif d'ostéosynthèse, la tige est mise en place par un geste opératoire très simple consistant en une simple translation de la tige à travers le reste du dispositif et vis-à-vis de l'organe d'ancrage de manière à assembler la tige à cet organe d'ancrage. Cela permet d'optimiser la section de la tige au niveau de sa partie insérée au sein du dispositif d'ostéosynthèse, tel qu'un clou centromédullaire, de manière à conserver un diamètre à ce niveau du clou parfaitement adapté au canal médullaire dans lequel il est inséré, et d'autre part d'optimiser la section de la tige de manière à ce que la vis osseuse, formée après assemblage de la tige et de l'organe d'ancrage, soit parfaitement stable et résistante.

Avantageusement, le dispositif d'ostéosynthèse présente au moins l'une des caractéristiques additionnelles suivantes.
- le dispositif comporte des moyens de clipsage aptes à renforcer la liaison par cônes Morse de la tige avec l'organe d'ancrage ;
- la partie distale de forme aplatie possède une section essentiellement rectangulaire dont les grands côtés sont matérialisés par deux méplats parallèles ;
- la partie proximale de ladite tige, qui est située entre sa partie distale et sa portion d'extrémité tronconique, a une forme cylindrique dont le diamètre est sensiblement égal à la distance entre les deux méplats ;
- sur l'une des faces reliant les deux méplats, la partie distale de ladite tige présente une rainure parallèle à l'axe principal de ladite tige, cette rainure étant adaptée pour recevoir l'extrémité d'une vis de serrage et de blocage traversant un orifice pratiqué axialement dans la partie proximale du clou et débouchant dans ledit orifice.

Dans une application possible de l'invention, le dispositif est destiné au traitement du fémur, et la vis est une vue céphalique dont l'organe d'ancrage est adapté pour être vissé dans la tête fémorale.

L'invention a également pour objet un dispositif d'ostéosynthèse qui comprend une plaque diaphysaire solidaire d'un canon, ainsi qu'une vis solidaire de ce canon et ladite vis est composée d'une tige et d'un organe d'ancrage ayant les caractéristiques énoncées plus haut, la partie distale de la tige étant adaptée pour être insérée à coulissement dans ledit canon, avec coopération de formes.

Avantageusement, ce dispositif comprend une vis qui permet de régler la position de ladite vis à l'intérieur du canon.

Le procédé chirurgical mettant en oeuvre le dispositif tel que défini ci-dessus comprend les étapes suivantes :
- réalisation dans un os tel que le fémur, à partir de l'épiphyse externe, d'un trou borgne d'axe sensiblement parallèle à l'axe du col de l'os et de dimensions légèrement inférieures aux dimensions de l'organe d'ancrage ;
- réalisation éventuelle d'un taraudage dans le trou borgne ;
- mise en place au fond du trou de l'organe d'ancrage du dispositif ; et
- insertion selon un mouvement de translation simple de la tige du dispositif dans le trou, puis assemblage avec l'organe d'ancrage.

D'autres caractéristiques, buts et avantages de l'invention apparaîtront lors de la description ci-après d'un mode préféré de réalisation ainsi que d'une variante. Sur les dessins annexés :
- les figures 1a, 1b et 1c sont des vues de dessous, de côté et de dessus respectivement d'une vis céphalique selon un premier mode de réalisation de l'invention ;
- la figure 2 est une vue en perspective éclatée de la vis céphalique des figures 1a à 1c ;
- la figure 3 est vue partielle en coupe selon la ligne A-A de la figure 2 ;
- la figure 4 est une vue en perspective d'un dispositif d'ostéosynthèse comprenant une vis selon les figures la à 3 et un clou médullaire ;
- la figure 5 est une vue en perspective éclatée du dispositif de la figure 4 ;
- la figure 6 est une vue en élévation de côté d'un dispositif d'ostéosynthèse comprenant une plaque et une vis selon l'invention.

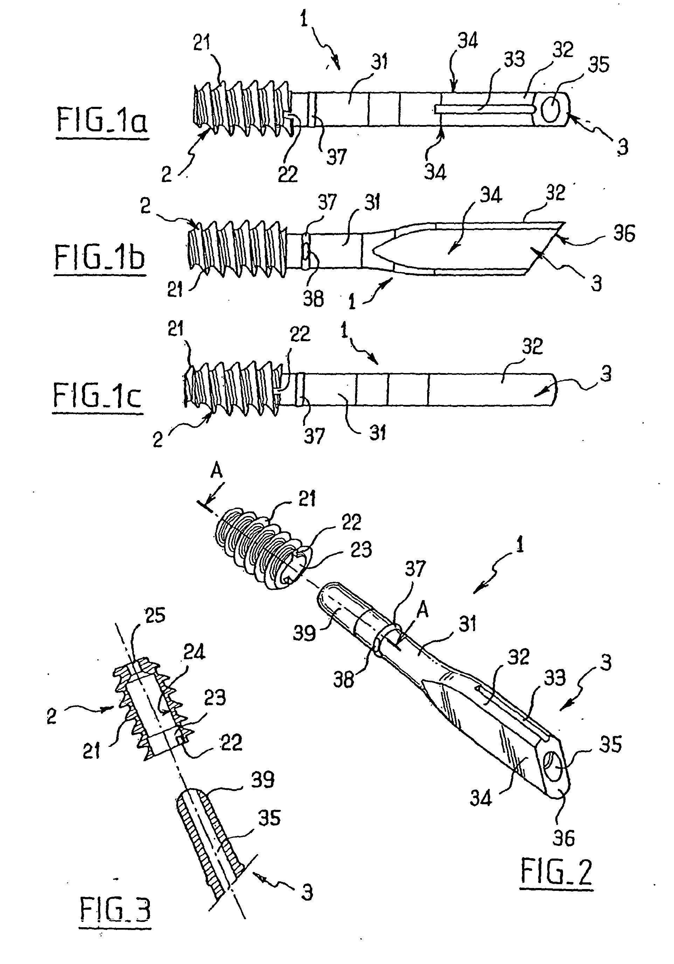

En référence tout d'abord aux figures la à 3, on va décrire un premier mode de réalisation préféré d'une vis osseuse de type céphalique selon l'invention. La vis céphalique comprend une tige 3 et un organe d'ancrage 2. La tige 3 est apte à être assemblée avec l'organe d'ancrage 2. L'organe d'ancrage 2 présente ici la forme d'un noyau essentiellement cylindrique de révolution comportant un filetage osseux externe 21. A une extrémité intérieure, le noyau présente une fente diamétrale 22 destinée à être utilisée lors d'une opération chirurgicale pour mettre en place le noyau 2 à l'aide d'un tournevis présentant une empreinte complémentaire. En référence à la figure 3, le noyau 22 présente un orifice axial traversant 23 présentant dans sa partie centrale une section tronconique 24 et, à l'extrémité opposée à l'extrémité présentant la fente 22, un orifice débouchant coaxial 25.

La tige 3 de la vis osseuse 1 présente une partie distale 32 et une partie proximale 31. La partie distale 32 est formée d'un cylindre de révolution tronqué par deux méplats 34 situés de part et d'autre d'un axe principal de la tige et parallèles l'un à l'autre. Entre les deux méplats 34, sur l'un des faces convexes reliant les deux méplats, la partie distale 32 présente une rainure 33 parallèle à l'axe principal de la tige et s'étendant sur une majeure partie de la partie distale 32. La partie distale 32 présente une extrémité distale 36 présentant une face plane préférentiellement non perpendiculaire à l'axe principal de la tige.

La partie proximale 31 de la tige 3 est de préférence cylindrique de révolution, avec un diamètre préférentiellement sensiblement égal à la distance séparant les deux méplats 34 de la partie distale 32. La partie proximale 31 présente un anneau circulaire 37 s'étendant en saillie radialement vers l'extérieur du cylindre formant la partie proximale 31, cet anneau 37 présentant deux méplats 38 situés de part et d'autre de l'axe principal de la tige et parallèles l'un à l'autre. De préférence, les deux méplats 38 sont parallèles aux deux méplats 34 de la partie distale 32. L'extrémité de la partie proximale 31 présente une partie tronconique 39 se rétrécissant en allant vers l'extrémité libre de la partie proximale 31. De préférence, le cône 39 est complémentaire du cône 24 formé dans le noyau 2. De ce fait, les deux cônes sont aptes à coopérer l'un avec l'autre de manière à lier ensemble le noyau et la tige pour former la vis osseuse 1, De manière préférentielle les cônes 39 et 24 sont des cônes Morse.

Il est à noter que la tige 3 présente un orifice 35 coaxial à son axe principal, partant de l'extrémité distale 36 et allant jusque l'extrémité libre de la partie proximale 31.

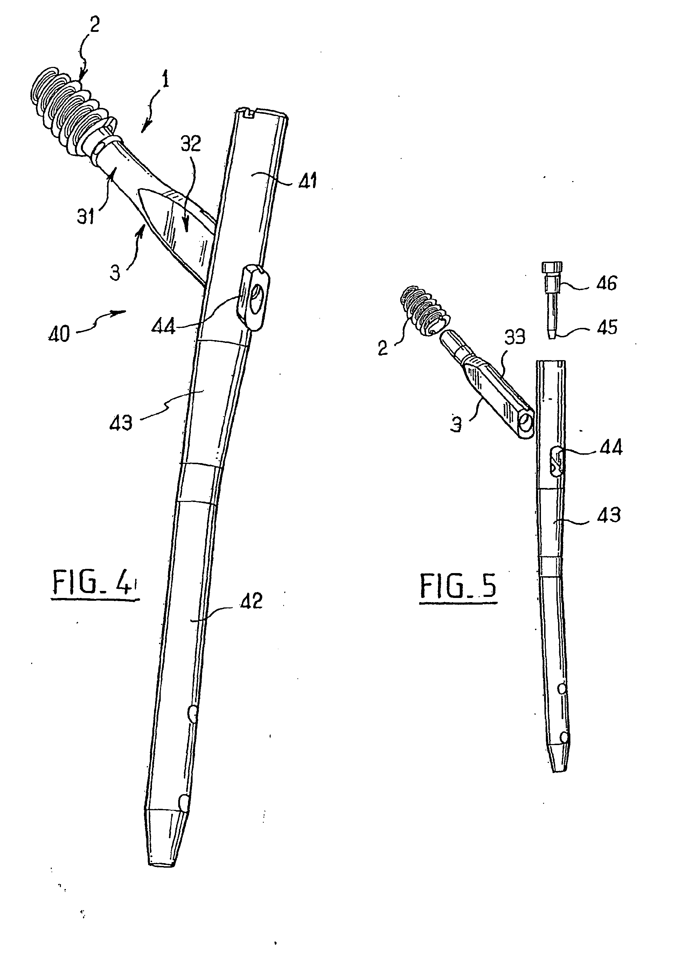

En référence aux figures 4 et 5, on va décrire l'utilisation de la vis osseuse de type céphalique 1 décrite ci-dessus dans le cadre d'un dispositif d'ostéosynthèse 40 qui comprend un clou centro-médullaire 43 présentant une partie distale 42 et une partie proximale 41. De manière préférentielle, le clou centro-médullaire 43 est un cylindre de révolution. La partie distale 42 présente un premier diamètre et la partie proximale 41 présente un deuxième diamètre sensiblement supérieur au diamètre de la partie distale 42. Dans la partie proximale 41 est aménagé un orifice traversant 44 dont la section est sensiblement identique à la section de la partie distale de la tige 3 de la vis céphalique 1. Ainsi, la tige peut être reçue à coulissement dans cet orifice 44 et les sections, ici sensiblement rectangulaire, de l'orifice 44 et de la partie distale 32 de la vis céphalique interdisent à cette dernière toute possibilité de rotation par rapport au clou centro-médullaire. De préférence, la tige est insérée dans l'orifice 44 du clou centromédullaire de manière à ce que la fente 33 de la tige soit tournée vers l'extrémité proximale du clou centro-médullaire. Cette fente 33 est apte à recevoir l'extrémité 45 d'une vis de serrage et de blocage 46 insérée dans un orifice pratiqué axialement dans la partie proximale 41 du clou, orifice s'étendant au moins depuis l'extrémité du clou jusque dans l'orifice 44 et agencé de manière à recevoir-la vis de blocage 46.

On va maintenant décrire les différentes opérations permettant la mise en place d'un tel dispositif d'ostéosynthèse au sein d'un fémur. Les étapes principales sont les suivantes :
- réalisation d'un accès au canal médullaire au niveau de la partie supérieur du fémur, entre le grand trochanter et le col fémoral et mise en place d'un clou fantôme, ou clou d'essai, dans ledit canal médullaire. Le clou fantôme est identique en forme au clou centromédullaire qui sera ultérieurement posé. Le clou fantôme est fendu de préférence depuis l'extrémité distal jusqu'à environ l'axe de l'orifice traversant 44. il permet de viser l'axe du col du fémur ;
- mise en place d'une broche de faible diamètre (de préférence de l'ordre de 2,5 mm environ) matérialisant l'axe du col de fémur ;
- retrait du clou fantôme en laissant en place la broche du fait que le clou fantôme est fendu comme précédemment décrit ;
- réalisation après un perçage sensiblement coaxial à l'axe du col du fémur en partant de l'épiphyse externe du fémur situé sous le grand trochanter jusqu'à environ le centre de la sphère formant la tête du fémur. Ce perçage est réalisé en enfilant un foret ou une tarière sur la broche précédemment installée ;
- réalisation éventuelle d'un taraudage du perçage ainsi réalisé ;
- mise en place du noyau 2 en l'enfilant sur la broche puis en vissant ledit noyau 2 au fond du perçage de manière à ce que les filets 21 soient pris dans l'os de la tête fémorale.
- après retrait de la broche, mise en place du clou centro-médullaire dans le canal médullaire du fémur de manière à ce que l'orifice traversant 44 du clou centro-médullaire se trouve dans l'axe du perçage ;
- mise en place sur la broche éventuellement remise en place comme précédemment décrit d'une tige 3 de manière à ce que la broche soit dans l'orifice traversant 35 de la tige 3 selon une translation simple jusqu'à ce que le cône 39 vienne en contact avec le cône 24, de manière à assembler le noyau 2 avec le canon 3 ;
- mise en place de moyens d'ancrage de la partie distale du clou dans le fémur ;
- retrait de la broche et installation de la vis 46 dans le clou centro-médullaire de manière à bloquer en position le canon 3 dans le clou 43.

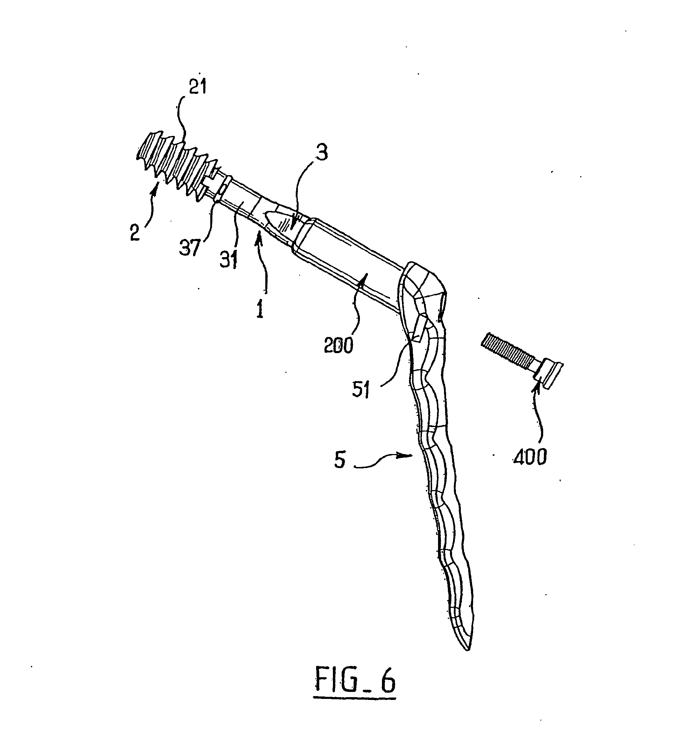

Maintenant en référence à la figure 6, on a représenté une autre forme de réalisation de l'invention dans une application dite de vis-plaque à compression, notamment pour la hanche.

Le dispositif comprend une plaque diaphysaire 5 solidaire d'un canon 200 apte à être assemblé avec la plaque 5 par tout moyen approprié, et la vis osseuse 1 comprenant ses deux parties 2 et 3, la partie 3 de section non circulaire étant insérée à coulissement dans le canon 200 avec coopération de formes. Le dispositif comprend enfin une vis de coaptation ou équivalente 400 apte à régler la position de la vis céphalique 1 au sein du canon 200. On notera ici que la plaque comporte des empreintes telles qu'une empreinte cylindrique 51 pour retenir la plaque sur un porte-plaque (non représenté) pendant la pose.

On pourra apporter à la présente invention de nombreuses modifications sans sortir du cadre de celle-ci. Notamment, il est possible de renforcer l'assemblage par cône Morse de la tige et du noyau par des moyens de clipsage. Ces moyens de clipsage peuvent se présenter sous la forme préférentielle d'une lèvre annulaire située de préférence au niveau du grand diamètre de la partie tronconique de la tige et dirigée de préférence vers la partie proximale de ladite tige. Cette lèvre est apte à coopérer avec au moins une languette déformable élastiquement, de préférence, située au niveau de l'extrémité 23 du noyau de manière préférentielle.

Dans une autre variante de réalisation, une fois l'assemblage réalisé, les moyens de fixation de la tige et du noyau peuvent permettre au moins une translation libre selon l'axe de la tige du noyau par rapport à la tige.

## Revendications

1. Dispositif d'ostéosynthèse, notamment métaphysaire, comprenant un clou centromédullaire (43) solidaire d'une vis (1) qui est composée de deux éléments distincts, à savoir une tige (3) et un organe d'ancrage (2), ce dernier consistant en un noyau essentiellement cylindrique comportant un filetage externe (21), apte à être mis en place par vissage dans l'os, tandis que la tige (3) est adaptée pour être insérée dans un orifice traversant (44) ménagé dans la partie proximale (41) du clou (43), **caractérisé par le fait que** :
a) ladite tige (3) possède une partie proximale (31) dont la portion d'extrémité (39) a une forme tronconique, se rétrécissant vers son extrémité libre, ainsi qu'une partie distale (32) de forme aplatie, à section allongée ;
b) ledit organe d'ancrage (2) possède un orifice axial (23) qui présente une section (24) de forme tronconique, complémentaire de ladite portion d'extrémité (39) de la partie proximale (31);
c) ledit orifice traversant (44) a la forme d'une lumière allongée, dont la grande direction est sensiblement parallèle à l'axe de la partie proximale (41) du clou (43), et dont la section est sensiblement identique à celle de la partie distale (32) de la tige (3), de sorte que cette dernière peut être reçue à coulissement dans ledit orifice (44), sans possibilité de rotation par rapport au clou centromédullaire (43);
d) l'assemblage de la tige (3) avec organe d'ancrage (2) est réalisé par simple translation de la tige (3) selon son axe longitudinal et engagement de sa portion d'extrémité (39) dans l'orifice axial (23) de l'organe d'ancrage (2), leur liaison mutuelle étant assurée par friction, par cônes Morse complémentaires.

2. Dispositif selon la revendication 1, **caractérisé par le fait qu'**il comporte des moyens de clipsage aptes à renforcer la liaison par cônes Morse de la tige (3) avec l'organe d'ancrage (2).

3. Dispositif selon la revendication 1 ou 2, **caractérisé par le fait que** ladite partie distale (32) de forme aplatie possède une section essentiellement rectangulaire dont les grands côtés sont matérialisés par deux méplats parallèles (34).

4. Dispositif selon la revendication 3, **caractérisé par le fait que** la partie proximale (31) de ladite tige (3), qui est située entre sa partie distale (32) et sa portion d'extrémité tronconique (39), a une forme cylindrique dont le diamètre est sensiblement égal à la distance entre les deux méplats (34).

5. Dispositif selon la revendication 4, **caractérisé par le fait que**, sur l'une des faces reliant les deux méplats (34), la partie distale (32) de ladite tige (3) présente une rainure (33) parallèle à l'axe principal de ladite tige (3), cette rainure étant adaptée pour recevoir l'extrémité (45) d'une vis de serrage et de blocage (46) traversant un orifice pratiqué axialement dans la partie proximale (41) du clou et débouchant dans ledit orifice (44).

6. Dispositif selon l'une des revendications 1 à 5, destiné au traitement du fémur, ladite vis (1) étant une vis céphalique dont l'organe d'ancrage (2) est adapté pour être vissé dans la tête fémorale.

7. Dispositif d'ostéosynthèse, qui comprend une plaque diaphysaire (5) solidaire d'un canon (200), ainsi qu'une vis (1) solidaire dudit canon (200), **caractérisé par le fait que**,
d'une part, cette vis (1) est composée de deux éléments distincts, à savoir une tige (3) et un organe d'ancrage (2), ce dernier consistant en un noyau essentiellement cylindrique comportant un filetage externe (21), apte à être mis en place par vissage dans l'os, tandis que la tige (3) est adaptée pour être insérée dans un orifice traversant (44) ménagé dans la partie proximale (41) du clou (43), ces éléments étant ainsi conformés et agencés tels que :
a) ladite tige (3) possède une partie proximale (31) dont la portion d'extrémité (39) a une forme tronconique, se rétrécissant vers son extrémité libre, ainsi qu'une partie distale (32) de forme aplatie, à section allongée ;
b) ledit organe d'ancrage (2) possède un orifice axial (23) qui présente une section (24) de forme tronconique, complémentaire de ladite portion d'extrémité (39) de la partie proximale (31);
c) ledit orifice traversant (44) a la forme d'une lumière allongée, dont la grande direction est sensiblement parallèle à l'axe de la partie proximale (41) du clou (43), et dont la section est sensiblement identique à celle de la partie distale (32) de la tige (3), de sorte que cette dernière peut être reçue à coulissement dans ledit orifice (44), sans possibilité de rotation par rapport au clou centromédullaire (43);
d) l'assemblage de la tige (3) avec organe d'ancrage (2) est réalisé par simple translation de la tige (3) selon son axe longitudinal et engagement de sa portion d'extrémité (39) dans l'orifice axial (23) de l'organe d'ancrage (2), leur liaison mutuelle étant assurée par friction, par cônes Morse complémentaires,
et que,
d'autre part, la partie distale (32) de ladite tige (3) est adaptée pour être insérée à coulissement dans ledit canon (200), avec coopération de formes.

8. Dispositif selon la revendication 7, **caractérisé par le fait qu'**il comprend une vis (400) permettant de régler la position de ladite vis (1) à l'intérieur du canon (200).

## Claims

1. An osteosynthesis device, in particular a metaphyseal device, comprising a centromedullary nail (43) secured to a screw (1) that is made up of two distinct elements, namely a shank (3) and an anchor member (2), the anchor member comprising an essentially cylindrical core carrying an outside thread (21), suitable for being put into place by being screwed into bone, while the shank (3) is adapted to be inserted in a through orifice (44) formed in the proximal portion (41) of the nail (43), the device being **characterized by** the facts that:
a) said shank (3) possesses a proximal portion (31) whose end portion (39) is frustoconical in shape, tapering towards its free end, and a distal portion (32) of flat shape with an elongate section;
b) said anchor member (2) possesses an axial orifice (23) that presents a section (24) of frustoconical shape that is complementary to said end portion (39) of the proximal portion (31);
c) said through orifice (44) is in the form of an elongate slot, having its long direction substantially parallel to the axis of the proximal portion (41) of the nail (43), and of section that is substantially identical to the distal portion (32) of the shank (3), thereby enabling the shank to be slidably received in said orifice (44), without any possibility of turning relative to the centromedullary nail (43); and
d) the shank (3) is assembled with the anchor member (2) merely by moving the shank (3) in translation along its longitudinal axis and engaging its end portion (39) in the axial orifice (23) of the anchor member (2), mutual cohesion therebetween being provided by friction, via complementary Morse tapers.

2. A device according to claim 1, **characterized by** the fact that it includes clip means suitable for reinforcing the Morse taper connection between the shank (3) and the anchor member (2).

3. A device according to claim 1 or claim 2, **characterized by** the fact that said distal portion (32) of flat shape possesses a section that is essentially rectangular, with its long sides being embodied by two parallel flats (34).

4. A device according to claim 3, **characterized by** the fact that the proximal portion (31) of said shank (3) that is situated between the distal portion (32) and the frustoconical end portion (39) thereof, is cylindrical in shape and of diameter that is substantially equal to the distance between the two flats (34).

5. A device according to claim 4, **characterized by** the fact that, on one of the faces interconnecting the two flats (34), the distal portion (32) of said shank (3) presents a groove (33) parallel to the main axis of said shank (3), said groove being adapted to receive the end (45) of a clamping and locking screw (46) passing through an orifice formed axially in the proximal portion (41) of the nail and opening out into said orifice (44).

6. A device according to any one of claims 1 to 5, for treating the femur, said screw (1) being a cephalic screw with its anchor member (2) being adapted to be screwed into the head of the femur.

7. An osteosynthesis device comprising a diaphyseal plate (5) secured to a rod (200) and a screw (1) secured to said rod (200), the device being **characterized by** the facts that, firstly,
said screw (1) is made up of two distinct elements, namely a shank (3) and an anchor member (2), the anchor member consisting in a core that is carrying an outside thread (21), suitable for being put into place by being screwed into bone, while the shank (3) is adapted to be inserted in a through orifice (44) formed in the proximal portion (41) of the nail (43), said elements thus being shaped and arranged such that:
a) said shank (3) possesses a proximal portion (31) whose end portion (39) is frustoconical in shape, tapering towards its free end, and a distal portion (32) of flat shape with an elongate section;
b) said anchor member (2) possesses an axial orifice (23) that presents a section (24) of frustoconical shape that is complementary to said end portion (39) of the proximal portion (31);
c) said through orifice (44) is in the form of an elongate slot, having its long direction substantially parallel to the axis of the proximal portion (41) of the nail (43), and of section that is substantially identical to the distal portion (32) of the shank (3), thereby enabling the shank to be slidably received in said orifice (44), without any possibility of turning relative to the centromedullary nail (43); and
d) the shank (3) is assembled with the anchor member (2) merely by moving the shank (3) in translation along its longitudinal axis and engaging its end portion (39) in the axial orifice (23) of the anchor member (2), mutual cohesion therebetween being provided by friction, via complementary Morse tapers;
and secondly,
the distal portion (32) of said shank (3) is adapted to be slidably inserted in said rod (200) with co-operating shapes.

8. A device according to claim 7, **characterized by** the fact that it includes a screw (400) suitable for adjusting the position of said screw (1) within the rod (200).

## Patentansprüche

1. Osteosynthesevorrichtung, besonders im Bereich der Metaphyse, umfassend einen durch die Mitte des Knochenmarks gehenden Nagel (43), der fest mit einer Schraube (1) verbunden ist, die aus zwei unterschiedlichen Elementen zusammengesetzt ist, und zwar einer Stange (3) und einem Verankerungsorgan (2), wobei letzteres aus einem im wesentlichen zylindrischen Kern besteht, der ein äußeres Gewinde (21) aufweist, das dazu geeignet ist, durch Einschrauben in den Knochen an Ort und Stelle verbracht zu werden, während die Stange (3) dazu ausgebildet ist, in eine Durchgangsöffnung (44) eingeführt zu werden, die in den proximalen Teil (41) des Nagels (43) eingebracht ist, **dadurch gekennzeichnet, dass**:
a) die genannte Stange (3) einen proximalen Teil (31), dessen Endabschnitt (39) eine Kegel- oder Kegelstumpfform aufweist, die sich nach ihrem freien Ende hin verjüngt, sowie einen distalen Abschnitt (32) mit abgeflachter Form und länglichem Querschnitt besitzt;
b) das genannte Verankerungsorgan (2) eine axiale Öffnung (23) besitzt, die einen kegel- oder kegelstumpfförmigen Querschnitt (24) aufweist, der komplementär zum genannten Endabschnitt (39) des proximalen Teils (31) ist;
c) die genannte Durchgangsöffnung (44) die Form einer länglichen Aussparung hat, deren Hauptrichtung im wesentlichen parallel zur Achse des proximalen Teils (41) des Nagels (43) ist, und deren Querschnitt im wesentlichen identisch zu dem des distalen Abschnitts (32) der Stange (3) ist, so dass dieser letztgenannte zur Gleitbewegung in der genannten Öffnung (44) ohne Möglichkeit der Drehung bezüglich des durch die Mitte des Knochenmarks gehenden Nagels (43) aufgenommen werden kann; und
d) der Zusammenbau der Stange(3) mit dem Verankerungsorgan (2) durch einfache Translationsbewegung der Stange (3) längs ihrer Längsachse und Eingriff ihres Endabschnitts (39) in die axiale Öffnung (23) des Verankerungsorgans (2) vorgenommen wird, wobei ihre gegenseitige Verbindung durch Reibung durch komplementäre Morsekegel sichergestellt wird.

2. Vorrichtung nach Anspruch 1, **dadurch gekennzeichnet, dass** sie Rastmittel umfasst, die geeignet sind, die Verbindung der Stange (3) mit dem Verankerungsorgan (2) durch Morsekegel zu verstärken.

3. Vorrichtung nach Anspruch 1 oder 2, **dadurch gekennzeichnet, dass** der genannte distale Abschnitt (32) mit abgeflachter Form einen im wesentlichen rechteckigen Querschnitt besitzt, dessen große Seiten durch zwei parallele Abflachungen (34) verkörpert sind.

4. Vorrichtung nach Anspruch 3, **dadurch gekennzeichnet, dass** der proximale Abschnitt (31) der genannten Stange (3), der zwischen ihrem distalen Abschnitt (32) und ihrem kegelstumpfförmigen Endabschnitt (39) gelegen ist, eine zylindrische Form hat, deren Durchmesser im wesentlichen gleich dem Abstand zwischen den beiden Abflachungen (34) ist.

5. Vorrichtung nach Anspruch 4, **dadurch gekennzeichnet, dass** auf der einen der Flächen, die die beiden Abflachungen (34) verbinden, der distale Abschnitt (32) der genannten Stange (3) eine Rille (33) aufweist, die parallel zur Hauptachse der genannten Stange (3) ist, wobei diese Rille dazu ausgebildet ist, das Ende (45) einer Klemm- und Blockierschraube (46) aufzunehmen, die eine Öffnung durchsetzt, die axial in den proximalen Teil (41) des Nagels eingebracht ist und in die genannte Öffnung (44) mündet.

6. Vorrichtung nach einem, der Ansprüche 1 bis 5, bestimmt zur Behandlung des Oberschenkelknochens, wobei die genannte Schraube (1) eine Kopfschraube ist, deren Verankerungsorgan (2) geeignet ist, in den Oberschenkelkopf geschraubt zu werden.

7. Osteosynthesevorrichtung, die eine Diaphyseplatte (5) umfasst, die fest mit einem Zylinder (200) verbunden ist, sowie eine Schraube (1), die fest mit dem genannten Zylinder (200) verbunden ist, **dadurch gekennzeichnet, dass** einerseits diese Schraube (1) zusammengesetzt ist aus zwei unterschiedlichen Elementen, und zwar einer Stange (3) und einem Verankerungsorgan (2), wobei letzteres aus einem im wesentlichen zylindrischen Kern besteht, der ein Außengewinde (21) umfasst, das geeignet ist, durch Verschrauben mit dem Knochen an Ort und Stelle verbracht zu werden, während die Stange (3) dazu ausgebildet ist, in eine Durchgangsöffnung (44) eingeführt zu werden, die im proximalen Teil (41) des Nagels (43) eingebracht ist, wobei diese Elemente so geformt und eingerichtet sind, dass:
a) die genannte Stange (3) einen proximalen Abschnitt (31), dessen Endabschnitt (39) eine Kegel- oder Kegelstumpfform aufweist, die sich zu seinem freien Ende hin verjüngt, sowie einen distalen Abschnitt (32) mit abgeflachter Form und länglichem Querschnitt besitzt;
b) das genannte Verankerungsorgan (2) eine axiale Öffnung (23) besitzt, die einen kegel- oder kegelstumpfförmigen Querschnitt (24) aufweist, der komplementär zum genannten Endabschnitt (39) des proximalen Teils (31) ist;
c) die genannte Durchgangsöffnung (44) die Form einer länglichen Aussparung hat, deren Hauptrichtung im wesentlichen parallel zur Achse des proximalen Teils (41) des Nagels (43) ist und deren Querschnitt im wesentlichen identisch zu dem des distalen Abschnitts (32) der Stange (3) ist, so dass diese letztgenannte zur Gleitbewegung in der genannten Öffnung (44) ohne die Möglichkeit der Drehung bezüglich des durch die Mitte des Knochenmarks gehenden Nagels (43) aufgenommen werden kann; und
d) der Zusammenbau der Stange (3) mit dem Verankerungsorgan (2) durch einfache Translationsbewegung der Stange (3) längs ihrer Längsachse und Eingriff ihres Endabschnitts (39) in die axiale Öffnung (23) des Verankerungsorgans (2) durchgeführt wird, wobei ihre gegenseitige Verbindung durch Reibung durch komplementäre Morsekegel sichergestellt wird,
und dass,
andererseits der distale Abschnitt (32) der genannten Stange (3) dazu ausgebildet ist, zur Gleitbewegung in den genannten Zylinder (200) mit Zusammenwirkung der Formen eingeführt zu werden.

8. Vorrichtung nach Anspruch 7, **dadurch gekennzeichnet, dass** sie eine Schraube (400) umfasst, die es gestattet, die Position der genannten Schraube (1) im Inneren des Zylinders (200) einzustellen.
